# EUROPÄISCHE PATENTANMELDUNG

(11) **EP 1 346 681 A2**
(43) Veröffentlichungstag der Anmeldung: **24.09.2003**
(21) Anmeldenummer: 03002889.8
(22) Anmeldetag: 10.02.2003
(51) Int. Cl.: A61B 1/12

(54) **Absaugventil für ein Endoskop**

(30) Priorität: 01.03.2002 DE 10209124
(71) Anmelder: Richard Wolf GmbH, 75438 Knittlingen (DE)
(72) Erfinder: Heimberger, Rudolf, 75038 Oberderdingen (DE)
(74) Vertreter: Hemmer, Arnd, Dipl.-Ing.

(57) **Zusammenfassung**

Die Erfindung betrifft ein Absaugventil für ein Endoskop, bei welchem ein Ventilgehäuse (2) mit einer Eintrittsöffnung (6) zum Verbinden mit einem Absaugkanal eines Endoskops vorgesehen ist, im Inneren des Ventilgehäuses (2) über der Eintrittsöffnung (6) eine geschlitzte Dichtmembran (14) angeordnet ist, die Dichtmembran (14) in dem Ventilgehäuse (2) durch einen Ventileinsatz (4) fixiert wird, in dem Ventileinsatz (4) ein quer zu der Dichtmembran (14) bewegbarer Ventilstößel (26) zum Öffnen der Dichtmembran (14) angeordnet ist, und der Ventileinsatz (4) kraft- und/oder formschlüssig in dem Ventilgehäuse (2) fixiert ist, sowie ein Endoskop mit einem solchen Absaugventil und einen Ventileinsatz für ein solches Absaugventil.

## Beschreibung

Die Erfindung betrifft ein Absaugventil für ein Endoskop, ein Endoskop mit einem solchen Absaugventil sowie einen zugehörigen Ventileinsatz.

Absaugventile, welche manuell über eine Drucktaste betätigt werden können, werden beispielsweise in Endoskopen, Fiberskopen oder anderen medizinischen Instrumenten eingesetzt, um ein Absaugen von Körpersekreten, Spülflüssigkeit oder dergleichen aus einer Körperhöhle zu ermöglichen.

Absaugventile, wie sie beispielsweise aus DE 29 54 069 C2 und EP O 106 310 B1 bekannt sind, weisen im Allgemeinen jedoch einen aufwändigen Aufbau auf. Diese Ventile müssen sowohl gas- als auch flüssigkeitsdicht ausgebildet sein. Die Ventile weisen kolbenartig ausgebildete Betätigungselemente auf, welche gegenüber dem Gehäuse durch O-Ringe oder dergleichen abgedichtet sind. Diese O-Ringe sind auf den kolbenartig ausgebildeten Betätigungselementen angeordnet und werden gemeinsam mit diesen axial innerhalb des Ventilgehäuses verschoben. Nachteilig ist bei dieser Anordnung eine durch Reibung verursachte Schwergängigkeit und durch Abnutzung verursachte Undichtigkeit der Ventile. Ein weiterer Nachteil besteht bei diesen aufwändig gestalteten Ventilen darin, dass sie konstruktionsbedingt nur unzureichend und umständlich zu reinigen sind. Ferner sind sie nur kosten- und zeitintensiv zu fertigen.

Es ist daher Aufgabe der Erfindung, ein vereinfachtes Absaugventil zu schaffen, welches kostengünstiger herzustellen und leichter zu reinigen ist. Ferner ist es Aufgabe der Erfindung, ein Endoskop mit einem derartig vereinfachten Absaugventil bereitzustellen.

Diese Aufgaben werden durch ein Absaugventil mit den im Anspruch 1 angegebenen Merkmalen, durch ein Endoskop mit den im Anspruch 18 angegebenen Merkmalen sowie durch einen Ventileinsatz für ein Absaugventil mit den im Anspruch 20 angegebenen Merkmalen gelöst. Bevorzugte Ausführungsformen ergeben sich aus den zugehörigen Unteransprüchen.

Das erfindungsgemäße Absaugventil ist angepasst an ein Endoskop ausgestaltet, wobei im Sinne der Erfindung auch andere medizinische Geräte, welche ein manuelles Öffnen einer Fluidleitung erfordern, beispielsweise Fiberskope, als Endoskope angesehen werden. Das erfindungsgemäße Absaugventil weist ein Ventilgehäuse auf, in dem eine Eintrittsöffnung zum Verbinden mit einem Absaugkanal eines Endoskops ausgebildet ist. Durch die Eintrittsöffnung tritt ein abzusaugendes bzw. ein zu förderndes Fluid in das Absaugventil ein. Im Inneren des Ventilgehäuses ist über der Eintrittsöffnung eine geschlitzte Dichtmembran angeordnet. Diese Dichtmembran dichtet die vor der Eintrittsöffnung liegende Absaugleitung vorzugsweise flüssigkeits- und druckdicht ab. Zum Öffnen wird die Dichtmembran im Bereich des Schlitzes aufgeweitet, insbesondere aufgeklappt, um einen Flüssigkeitseintritt in das Absaugventil zu ermöglichen. Die Dichtmembran ist in dem Ventilgehäuse durch einen Ventileinsatz fixiert. In dem Ventileinsatz ist ein quer, d.h. insbesondere senkrecht zu der Dichtmembran bewegbarer Ventilstößel zum Öffnen der Dichtmembran angeordnet. Zum Öffnen drückt der Ventilstößel gegen die Dichtmembran, so dass die Bereiche angrenzend an den Schlitz ausgelenkt bzw. aufgeklappt werden, so dass eine Öffnung in der Dichtmembran freigegeben wird. Dabei werden die Abschnitte der Dichtmembran vorzugsweise entgegen der Strömungsrichtung des abzusaugenden Fluids ausgelenkt. Im geschlossenen Zustand liegt die Dichtmembran bevorzugt an der Stirnseite des Ventileinsatzes bzw. des Ventilstößels an. Dies hat den Vorteil, dass die beweglichen Teile der Dichtmembran durch den Fluiddruck im geschlossenen Zustand des Ventils gegen den Ventilstößel gedrückt werden. Durch den Ventileinsatz bzw. den Ventilstößel wird dabei eine Auslenkung der beweglichen Teile der Dichtmembran in Strömungsrichtung des Fluids verhindert, so dass das Ventil ein druckdichtes Verschließen einer Absaugleitung ermöglicht. Die Dichtmembran ist bevorzugt elastisch, beispielsweise aus einem Polymer, ausgebildet. Dies bewirkt, dass die Dichtmembran bei ihrem Öffnen bzw. Auslenken eine Rückstellkraft aufbaut, welche beim Lösen des Ventilstößels diesen wieder zurück in seine Ausgangslage bewegt. Dies ermöglicht, auf zusätzliche Rückstellelemente, beispielsweise Federn, zu verzichten. Der Ventileinsatz, welcher dazu dient, den Ventilstößel zu halten und zu führen, ist kraftund/oder formschlüssig in dem Ventilgehäuse fixiert. Auf diese Weise übernimmt der Ventileinsatz eine Doppelfunktion. Zum einen führt er den Ventilstößel und zum anderen fixiert er die Dichtmembran innerhalb des Ventilgehäuses. Dies ermöglicht einen insgesamt sehr einfachen Aufbau des Absaugventils, da im Wesentlichen nur vier Bauteile erforderlich sind, nämlich das Ventilgehäuse, der Ventileinsatz mit dem Ventilstößel und die Dichtmembran. Dies ermöglicht eine einfache und kostengünstige Fertigung und erlaubt eine leichte Zerlegbarkeit des Ventils zu Reinigungszwecken.

Zweckmäßigerweise ist an dem Ventileinsatz ein Auslassstutzen ausgebildet, welcher mit dem Ventilgehäuse zur Fixierung des Ventils formschlüssig in Eingriff ist. Der Anschlussstutzen dient dazu, an das Ventil eine Absaug- bzw. Fluidleitung anzuschließen, durch die das Fluid weitergeleitet wird. Der Anschlussstutzen übernimmt eine zusätzliche Funktion, er fixiert den Ventileinsatz in dem Ventilgehäuse. Vorzugsweise ist der Auslassstutzen dazu lösbar mit dem Ventilgehäuse in Eingriff bringbar, so dass der Ventileinsatz leicht wieder von dem Gehäuse gelöst werden kann, um das gesamte Absaugventil, beispielsweise zu Reinigungszwecken, zu zerlegen. Ferner ermöglicht diese Anordnung einen leichten Austausch der Dichtmembran, da diese erfindungsgemäß in dem Ventilgehäuse lediglich durch den Ventileinsatz fixiert wird.

Vorzugsweise weist das Ventilgehäuse einen zylindrischen, insbesondere kreiszylindrischen Innenraum auf, wobei die Eintrittsöffnung an einer ersten Stirnseite des Ventilgehäuses vorgesehen ist und die entgegengesetzte Stirnseite des Ventilgehäuses zur Aufnahme des Ventileinsatzes offen ausgebildet ist und der Ventileinsatz eine zu dem Innenraum korrespondierende zylindrische, insbesondere kreiszylindrische Außenkontur aufweist. Diese Ausgestaltung ermöglicht, dass der Ventileinsatz passend in dem Ventilgehäuse gehalten werden kann. Durch die offen ausgebildete Seite des Ventilgehäuses kann der Ventileinsatz in Längsrichtung des Ventilgehäuses in dieses eingesetzt werden. Die kreiszylindrische Ausgestaltung ermöglicht ein Verdrehen des Ventileinsatzes in dem Ventilgehäuse zu dessen Sicherung.

Weiter bevorzugt weist auch die Dichtmembran eine dem Querschnitt des Innenraumes entsprechende Außenkontur auf und erstreckt sich quer zur Längsachse des Ventilgehäuses. Diese Ausgestaltung ermöglicht, dass die Dichtmembran im Wesentlichen an der einen Stirnseite des Ventileinsatzes anliegt. Ferner wird sie in radialer Richtung in dem Innenraum fixiert, da sie eine Außenkontur, insbesondere einen Außendurchmesser aufweist, welcher der Innenkontur bzw. dem Innendurchmesser des Ventilgehäuses entspricht.

Die Dichtmembran weist bevorzugt zumindest zwei sich kreuzende Schlitze auf, welche sich in Dickenrichtung durch die Dichtmembran hindurch erstrecken. Dies bedeutet, die Schlitze verbinden die beiden planen und vorzugsweise zueinander parallelen Oberflächen der Dichtmembran miteinander. Die gekreuzte Anordnung von zwei Schlitzen erreicht, dass im Zentrum bzw. im Inneren der Dichtmembran vier dreieckige bzw. viertelkreisförmige, bewegliche Abschnitte geschaffen werden. Diese beweglichen Abschnitte dienen als Klappen, welche ausgelenkt werden, um die Dichtmembran bzw. das Absaugventil zu öffnen. Der Ventilstößel drückt dazu gegen diese von den gekreuzten Schlitzen begrenzten, beweglichen Teile der Dichtmembran und lenkt diese aus der Ebene der Membran aus, wodurch eine Öffnung freigegeben wird. Bei elastischer Ausgestaltung der Membran bewegen sich diese beweglichen Abschnitte bzw. Klappen beim Lösen des Druckes durch den Ventilstößel in ihre Ausgangslage zurück. Dabei stoßen die Klappen mit ihren Stirnkanten, d. h. den Seitenflächen der Schlitze aneinander an und dichten den Durchgang vollständig ab.

In einer bevorzugten Ausführungsform weist die Dichtmembran in ihrem Zentrum eine größere Dicke auf als in ihrem Umfangsbereich. Diese Ausgestaltung schafft eine größere Festigkeit der Dichtmembran, was zu einer verbesserten Abdichtung führt. Ferner kann eine größere Rückstellkraft der Membran bzw. der beweglichen Dichtmembranteile erreicht werden.

Weiter bevorzugt ist zumindest der Mittelbereich der Dichtmembran kegelförmig ausgebildet. Bei dieser Ausgestaltung ist vorzugsweise eine Seite der Membran plan ausgebildet, während die andere sich zur Mitte der Dichtmembran kegelförmig erhebt. Dabei ist die plane Seite der Membran zweckmäßigerweise dem Ventilstößel zugewandt, um im geschlossenen Zustand mit diesem zur Anlage zu kommen.

Der Kreuzungspunkt der zumindest zwei Schlitze in der Dichtmembran liegt zweckmäßigerweise in dem Bereich der größten Dicke der Dichtmembran. Bei dieser Anordnung bewirkt die Ausgestaltung der Dichtmembran mit zunehmender Dicke, insbesondere die kegelförmige Ausgestaltung der Dichtmembran, dass die in den Schlitzen aneinander anliegenden Seitenflächen in Dickenrichtung der Membran eine möglichst große Länge aufweisen. Auf diese Weise wird zwischen den beweglichen Abschnitten der Membran eine möglichst große Anlagefläche geschaffen, um eine zuverlässigere Abdichtung zu erreichen. Der schräge bzw. kegelige Verlauf der Membrandicke ist zweckmäßigerweise so gewählt, dass die Membran in dem Bereich die geringste Dicke aufweist, in dem die Verformung zum Auslenken bzw. Aufklappen der Membran erfolgt. Damit können die Betätigungskräfte zum Öffnen des Absaugventils, d. h. zum Bewegen der beweglichen Membranteile gering gehalten werden.

In einer weiteren bevorzugten Ausführungsform ist an der dem Ventilstößel zugewandten Oberfläche der Dichtmembran eine zentrische, vorzugsweise kegelförmige Ausnehmung ausgebildet. Diese Ausnehmung erstreckt sich in das Innere der Dichtmembran hinein, jedoch nicht durch diese hindurch. Die Ausnehmung bewirkt eine Vergrößerung der beim Öffnen des Ventils geschaffenen Durchtrittsöffnung, da aufgrund der schrägen bzw. kegeligen Ausgestaltung der Ausnehmung weniger Material in die Durchtrittsöffnung und damit in den Strömungsweg hineinragt. Auf diese Weise kann bei minimalen Hub des Ventilstößels eine große Durchtrittsöffnung geschaffen werden.

Die Dichtmembran ist bevorzugt in ihrem Umfangsbereich zwischen einem Absatz in dem Ventilgehäuse und einer Stirnseite des Ventileinsatzes eingeklemmt. Hierzu kann in dem Ventilgehäuse ein ringförmiger Absatz bzw. eine ringförmige Schulter ausgebildet sein, an der die Membran in ihrem gesamten Umfangsbereich zur Anlage kommt, so dass eine sichere Abdichtung zwischen Dichtmembran und Ventilgehäuse erreicht wird. An ihrer entgegengesetzten Seite liegt die Dichtmembran ebenfalls vorzugsweise mit ihrem gesamten Umfangsbereich an der Stirnseite des Ventileinsatzes an, so dass eine gleichförmige Andruckkraft auf die Dichtmembran wirkt. Der innere Bereich bzw. Mittelbereich der Dichtmembran ist vorzugsweise frei durch den Ventilstößel beweglich, um ein leichtes Öffnen und Schließen der Dichtmembran zu ermöglichen. Zweckmäßigerweise ist das Ventilgehäuse napfförmig ausgebildet, wobei im Boden die Eintrittsöffnung angeordnet ist. Im Umfang des Innenraums ist eine Anlageschulter ausgebildet, an welcher die Dichtmembran anliegt, wenn der Ventileinsatz in das napfförmige Ventilgehäuse eingesetzt und mit diesem verbunden wird, so dass die Dichtmembran am Boden des Ventilgehäuses über der Eintrittsöffnung fixiert ist. Es sind somit keine zusätzlichen Befestigungselemente zum Fixieren der Dichtmembran erforderlich.

Ferner kann zusätzlich an der dem Ventilstößel zugewandten Oberfläche der Dichtmembran eine Ringnut ausgebildet sein, welche radial außerhalb der Schlitze verläuft. Die Schlitze sind somit vollständig innerhalb der von der Ringnut umschlossenen Fläche angeordnet. Die Ringnut dient dazu, die elastisch auslenkbaren Klappen zwischen den Schlitzen von dem zwischen dem Gehäuse und dem Ventileinsatz eingespannten Randbereich der Dichtmembran zu entkoppeln. Durch diese so genannte Entkopplung wird erreicht, dass die beim Einfügen des Ventileinsatzes in das Ventilgehäuse durch Reibung einerseits und durch Stauchung im Klemmbereich andererseits verursachten elastischen Verformungen der Dichtmembran nicht auf die von den Schlitzen begrenzten Klappen übertragen werden. Dies bedeutet, dass beim Einsetzen und Einklemmen der Dichtmembran eine Verformung der Klappen sowie eine Änderung der zwischen den Klappen befindlichen Schlitze und somit eine negative Beeinträchtigung der Dichtwirkung verhindert wird.

Der Auslassstutzen erstreckt sich bevorzugt quer zur Längsachse des Ventilgehäuses und der Eintrittsöffnung. Das heißt, der Auslassstutzen erstreckt sich radial von dem Ventileinsatz weg. Diese Anordnung ermöglicht, einen Ablaufschlauch so an das Ventil anzuschließen, dass er einen Operateur bei der Betätigung des Absaugventils wenig behindert. Ferner eignet sich ein solcher Auslassstutzen dazu, gleichzeitig den Ventileinsatz in dem Ventilgehäuse zu fixieren. Eine Fixierung des Ventileinsatzes in dem Ventilgehäuse ist insbesondere in Längsrichtung, d. h. in der Betätigungsrichtung des Ventilstößels erforderlich. Um diese Fixierung zu erreichen, kann der Auslassstutzen in eine entsprechende Ausnehmung in dem Ventilgehäuse eingehakt werden bzw. einen entsprechenden Vorsprung in dem Ventilgehäuse hintergreifen. Auf diese Weise kann auf zusätzliche Befestigungselemente zum Fixieren des Ventileinsatzes in dem Ventilgehäuse verzichtet werden, wodurch die Anzahl der erforderlichen Einzelteile minimiert und die Herstellung vereinfacht wird. Beispielsweise kann der Ventileinsatz mit dem abgewinkelten Auslassstutzen nach Art eines Bajonettverschlusses mit dem Ventilgehäuse verbunden werden, indem der Ventileinsaiz zunächst in Richtung der Längsachse des Ventilgehäuses bewegt und dann um diese Achse verdreht wird.

Hierzu ist vorzugsweise in einer Wandung des Ventilgehäuses eine L-förmige Nut ausgebildet, deren erster Abschnitt sich im Wesentlichen parallel zur Längsachse des Ventilgehäuses erstreckt und zum Rand des Ventilgehäuses hin geöffnet ist und deren zweiter Abschnitt quer zu dem ersten Abschnitt im Wesentlichen in Umfangsrichtung des Ventilgehäuses verläuft, wobei die Nut eine Breite aufweist, welche der Querschnittsgröße, d. h. vorzugsweise dem Durchmesser des Auslassstutzens entspricht. Bei einem solchen Ventilgehäuse wird der Ventileinsatz mit dem abgewinkelten Auslassstutzen so in Richtung der Längsachse des Ventilgehäuses eingesetzt, dass der Auslassstutzen zunächst durch den zur Längsachse parallelen Teil bzw. Schenkel der Nut bewegt wird. Wenn der Ventileinsatz weit genug, d. h. vorzugsweise in Längsrichtung vollständig in das Ventilgehäuse eingesetzt ist, hat der Auslassstutzen den Anfang des in Umfangsrichtung verlaufenden Nut-Abschnittes erreicht. Dies ermöglicht, dass der Ventileinsatz nun um die Längsachse des Ventils bzw. des Ventilgehäuses gedreht werden kann, wobei sich der Auslassstutzen in seiner Winkelposition bezüglich der Längsachse durch den in Umfangsrichtung verlaufenden Teil der L-förmigen Nut bewegt. Wenn der Auslassstutzen vollständig in diesem quer bzw. in Umfangsrichtung verlaufenden Nutteil liegt, ist der Ventileinsatz in Richtung der Längsachse in dem Ventilgehäuse gesichert. Um ein versehentliches Zurückdrehen des Ventileinsatzes zu verhindern, kann in der L-förmigen Nut in dem in Umfangsrichtung verlaufenden Schenkel eine zusätzliche Ausnehmung bzw. Aussparung ausgebildet sein, in die der Auslassstutzen eingreift, um eine Verdrehung zu verhindern. Diese Sicherung wird idealerweise durch die elastische Ausgestaltung der Dichtmembran unterstützt. Beim Einsetzen des Ventileinsatzes in das Ventilgehäuse wird die Dichtmembran durch den Ventileinsatz gegen die Ring- bzw. Anlageschulter im Inneren des Ventilgehäuses gedrückt, so dass die Dichtmembran zunächst geringfügig gestaucht wird. Erreicht der Auslassstutzen seine endgültige Position in der L-förmigen Nut, d. h. den Bereich einer zusätzlich vorgesehenen Aussparung entspannt sich die Dichtmembran zumindest teilweise und drückt den Auslassstutzen in die Aussparung, wodurch eine zusätzliche, gegen die Federwirkung der Dichtmembran lösbare Sicherung erreicht wird.

Der Ventileinsatz ist bevorzugt derart ausgebildet, dass er in Richtung der Längsachse des Ventilgehäuses ein Durchgangsloch bzw. einen Innenraum, in welchem der Ventilstößel in Längsrichtung beweglich angeordnet ist, und eine Austrittsöffnung aufweist, welches sich in radialer Richtung zu dem Durchgangsloch hin öffnet, wobei der Ventilstößel rohrförmig ausgebildet ist und an seiner der Dichtmembran zugewandten Seite eine Eintrittsöffnung und an seinem Umfang zumindest eine radiale Öffnung aufweist, welche mit der Austrittsöffnung in Verbindung steht. Zum Öffnen des Ventils wird der Ventilstößel gegen die Dichtmembran gedrückt, so dass deren bewegliche Teile ausgelenkt werden und eine Öffnung freigegeben wird. Dabei ist die Eintrittsöffnung des Ventilstößels der Öffnung in der Dichtmembran zugewandt, so dass ein Fluid in das Innere des Ventilstößels einströmen und weiter durch die radiale Öffnung in dem Ventilstößel zu der Austrittsöffnung und durch den Auslassstutzen strömen kann. Diese Anordnung ermöglicht eine sehr kompakt Ausgestaltung des Absaugventils, da der freizugebende Strömungsweg in das Innere des Ventilstößels gelegt ist. Zusätzlich kann der Innenraum des Ventilgehäuses im Umfang des Ventilstößels ringförmig erweitert sein, so dass ein ringförmiger Hohlraum gebildet wird, durch welchen ein Fluid von der radialen Öffnung in dem Ventilstößel zu der Austrittsöffnung strömen kann. Durch diese Anordnung kann auf einen genaue Positionierung der radialen Öffnung zu der Austrittsöffnung verzichtet werden.

Die der Dichtmembran abgewandte Stirnseite des Ventilstöβels ist vorzugsweise als geschlossene Druckkappe ausgebildet. Auf diese Weise wird verhindert, dass ein abzusaugendes Fluid an dieser Seite aus dem Ventilstößel austreten oder an dieser Stelle Umgebungsluft angesaugt werden kann. Die Druckkappe dient gleichzeitig zur Betätigung des Ventilstößels, indem er durch Fingerdruck in seiner Längsrichtung zum Öffnen der Dichtmembran bewegt werden kann. Um eine große Druckfläche und eine nach außen hin glatte Ausgestaltung des Absaugventils zu erreichen, überdeckt die Druckkappe an der Oberseite bevorzugt den gesamten Ventileinsatz.

Zusätzlich kann zwischen der Druckkappe und dem Ventileinsatz ein Dichtelement angeordnet sein, welches in geöffnetem Zustand des Absaugventils das Durchgangsloch bzw. den Innenraum in dem Ventileinsatz zu der Druckkappe hin abdichtet. Das Dichtelement ist vorzugsweise ein O-Ring, welcher den Ventilstößel angrenzend zu der Druckkappe umgibt. Wenn das Absaugventil in seiner geöffneten Position ist, d. h. der Ventilstößel vollständig in den Ventileinsatz hineingedrückt ist, kommt dieses Dichtelement bzw. der O-Ring mit der oberen bzw. äußeren Stirnseite des Ventileinsatzes bzw. der Umfangskante des Durchgangsloches des Ventileinsatzes zur Anlage und dichtet den Innenraum des Ventileinsatzes nach außen hin ab. Hierdurch wird erreicht, dass eine Abdichtung an dieser Stelle nur in geöffnetem Zustand des Absaugventils erfolgt. Befindet sich das Absaugventil in seiner geschlossenen Stellung, wird der Innenraum des Ventileinsatzes an seiner der Dichtmembran abgewandten Seite nicht durch das Dichtelement abgedichtet, so dass durch eine Absaugleitung, welche ständig mit Unterdruck beaufschlagt ist, "Falschluft" angesaugt werden kann.
Bevorzugt bildet der Ventileinsatz mit dem Ventilstößel und der Dichtmembran eine austauschbare Baugruppe. Diese Ausge- staltung ermöglicht, dass der gesamte Ventileinsatz als Verschleiß- bzw. Wegwerfteil ausgebildet werden kann, welches nach einmaligem Gebrauch ersetzt wird. Dies erspart eine aufwändige Reinigung des Absaugventils. Ferner kann der Ventileinsatz auch bei Verschleiß leicht ersetzt werden, ohne dass aufwändige Reparaturarbeiten erforderlich sind. Bevorzugt werden sämtliche Teile des Ventileinsatzes, d. h. ebenfalls der Ventilstößel und die Dichtmembran aus Kunststoff gefertigt und vormontiert, was eine kostengünstige Massenfertigung erlaubt.

Die Bewegungsmöglichkeit des Ventilstößels innerhalb des Ventileinsatzes wird dabei zweckmäßigerweise durch zumindest einen Anschlag begrenzt, d.h. vorzugsweise wird die Position des Ventilstößels in der oberen, d.h. unbetätigten Endstellung durch einen Anschlag begrenzt. Als zweiter Anschlag kann die Druckkappe dienen. Der erste Anschlag kann als vorzugsweise ringförmiger Rastvorsprung am Ventilstößel ausgebildet sein, welcher an einer entsprechenden nach innen gerichteten Ringschulter in dem Innenraum des Ventileinsatzes angreift. Ein solcher Anschlag dient dazu, den Ventilstößel sicher in dem Ventileinsatz zu halten, so dass dieser nicht unbeabsichligt aus diesem herausgezogen werden kann, was zu einem unbeabsichtigten Öffnen des Absaugventils während einer Operation führen könnte. Ferner kann der Ventilstößel auch während der Lagerung und Reinigung des Ventileinsatzes nicht verloren gehen. Der Anschlag bzw. Vorsprung an dem Ventilstößel kann jedoch dermaßen als Rastvorsprung ausgebildet sein, dass beim Aufbringen einer entsprechend größeren Zugkraft der Ventilstößel wieder aus dem Ventileinsatz entnommen werden kann, um die Teile z.B. reinigen oder austauschen zu können. Dazu kann der ringförmige Rastvorsprung an dem Ventilstößel beispielsweise mindestens eine, vorteilhaft jedoch mehrere schlitzförmige Öffnungen aufweisen.

Vorzugsweise ist der zumindest eine Anschlag auf einem an dem Ventilstößel ausgebildeten elastischen Steg angeordnet. Auf diese Weise kann der Anschlag einen elastischen Rastvorsprung bilden, welcher ein Einsetzen und Lösen des Ventilstößels in den bzw. von dem Ventileinsatz ermöglicht. Wenn die auf den Anschlag wirkende Kraft die Federkraft des Stegs übersteigt, wird der Anschlag entsprechend ausgelenkt, so dass er einen in dem Ventileinsatz ausgebildeten korrespondierenden Rastvorsprung bzw. die korrespondierende Ringschulter in dem Ventileinsatz passieren kann. Bevorzugt wird der elastische Steg von einem Wandabschnitt des Ventilstößels gebildet. Wenn in dem Ventilstößel mehrerer Durchgangslöcher ausgebildet sind, können derartige elastische Stege von den zwischen den Durchgangslöchern gelegenen Wandabschnitten gebildet werden. Die erforderliche Elastizität wird durch Dicke und Breite dieser Wandabschnitte eingestellt. Diese Ausgestaltung hat den Vorteil, dass keine zusätzlichen Federelemente eingesetzt werden müssen, da die erforderliche Federwirkung durch ohnehin vorhandene schmale Wandabschnitte zwischen den Durchgangslöchern gebildet werden.

Ferner ist zweckmäßigerweise am Umfang des Ventileinsatzes zumindest ein Vorsprung ausgebildet, welchen die Dichtmembran umgreift. Besonders bevorzugt ist hierzu am unteren der Dichtmembran zugewandten Ende des Ventileinsatzes an dessen Umfang eine Ringnut ausgebildet, in welche die Dichtmembran eingreift. Auf diese Weise kann die Dichtmembran an dem Ventileinsatz gehalten und gemeinsam mit diesem leicht ausgetauscht werden.

Das Ventilgehäuse kann fest mit dem Endoskop verbunden sein, während der Ventileinsatz mit dem Ventilstößel und der Dichtmembran austauschbar und aus dem Ventilgehäuse entnehmbar ausgebildet ist. So wird die Anzahl der Einzelteile, welche zur Reinigung zerlegt werden müssen, minimiert. Zur Reinigung ist es ausreichend, den Ventileinsatz aus dem Ventilgehäuse zu entnehmen. Anschließend kann der Ventileinsatz ebenfalls gereinigt werden oder durch einen neuen Ventileinsatz ersetzt werden. Wenn der Ventileinsatz beispielsweise aus Metall ist, so kann ggf. nur der Austausch der Dichtmembran erforderlich sein.

Weiter bevorzugt ist das Ventilgehäuse einstückig mit zumindest einem Bauteil des Endoskops ausgebildet, wodurch sich die Anzahl der erforderlichen und zu montierenden Einzelteile weiter reduziert.

Die Erfindung betrifft ferner einen zu dem zuvor beschriebenen Absaugventil zugehörigen Ventileinsatz, welcher beispielsweise in Form einer Baueinheit als Ersatzteil angeboten werden kann. Der Ventileinsatz weist ein in Längsrichtung verlaufendes Durchgangsloch bzw. Innenraum auf, in dem ein in Längsrichtung beweglicher Ventilstößel angeordnet ist, und einen sich radial erstreckenden Anschlussstutzen. Der Ventileinsatz kann gemeinsam mit dem Ventilstößel in ein Ventilgehäuse eingesetzt werden, wobei der sich radial erstreckende Anschlussstutzen zur bajonettverschlussartigen Verriegelung des Ventileinsatzes in dem Ventilgehäuse dient.

An einer Stirnseite des Ventileinsatzes ist vorzugsweise eine Dichtmembran befestigt, welche durch die Bewegung des Ventilstößels geöffnet werden kann. Das Öffnen und Schließen der Dichtmembran erfolgt in der oben beschriebenen Weise. Die feste Anordnung der Ventilmembran an dem Ventileinsatz bewirkt, dass sämtliche Verschleiß- und Austauschteile des Ventils gemeinsam mit dem Ventileinsatz in einer Baugruppe integriert sind, so dass ein leichter Austausch der Verschleißteile möglich ist.

Der Ventilstößel ist vorzugsweise rohrförmig ausgebildet und weist an einer Stirnseite eine Eintrittsöffnung sowie an seinem Umfang zumindest eine radiale Austrittsöffnung auf, welche mit dem Auslassstutzen in Verbindung steht. Das Innere des Ventilstößels kann so einen Strömungsweg bilden, so dass insgesamt eine kompakte Ausgestaltung des Ventileinsatzes und somit eines zugehörigen Absaugventils erreicht wird.

Die der Eintrittsöffnung entgegengesetzte Stirnseite des Ventilstößels ist bevorzugt als geschlossene Druckfläche ausgebildet. Diese Fläche dient zur Betätigung des Ventils und ist demgemäß zweckmäßigerweise besonders groß ausgebildet, um eine ausreichende Fingerauflage für eine sichere Betätigung des Ventils zu gewährleisten.

Nachfolgend wird die Erfindung beispielhaft anhand der beigefügten Figuren beschrieben. In diesen zeigt:
Fig. 1 eine Längsschnittansicht des erfindungsgemäßen Absaugventils in geschlossenem Zustand,
Fig. 2 eine Längsschnittansicht des erfindungsgemäßen Absaugventils in geöffnetem Zustand,
Fig. 3 eine Längsschnittansicht des Ventilgehäuses,
Fig. 4 eine perspektivische Gesamtansicht des erfindungsgemäßen Absaugventils,
Fig. 5 eine Draufsicht auf die Dichtmembran des erfindungsgemäßen Absaugventils, und
Fig. 6 eine Schnittansicht einer Dichtmembran gemäß einer speziellen Ausführungsform.

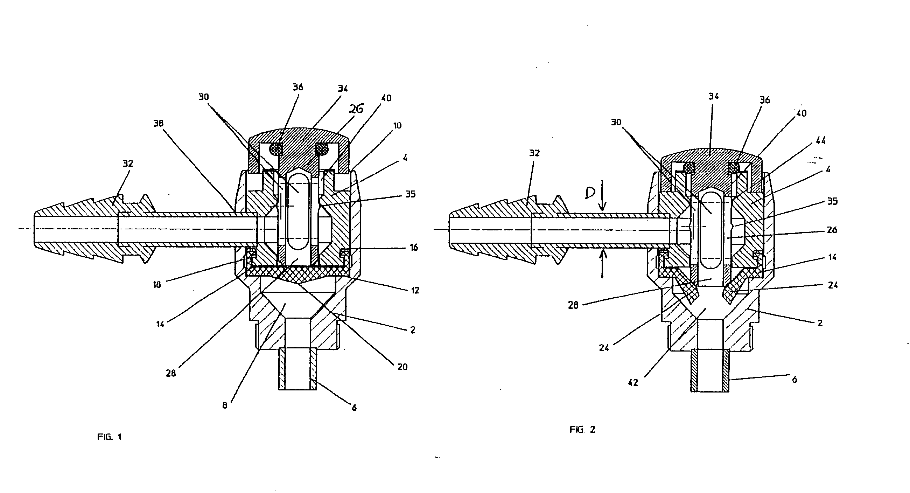

Fig. 1 zeigt das erfindungsgemäße Ventil in seinem unbetätigten Zustand, d. h. das Ventil ist druckdicht verschlossen. Das Ventil, welches vorzugsweise am proximalen Ende eines Endoskops (hier nicht gezeigt) angeordnet ist, weist ein Ventilgehäuse 2 mit einem darin angeordneten Ventileinsatz 4 auf. Das Ventilgehäuse 2 ist mit einem Endoskop verbunden oder einstückig mit diesem ausgebildet. Das Ventilgehäuse 2 ist im gezeigten Beispiel im Wesentlichen kreiszylindrisch ausgebildet und weist an seiner ersten Stirnseite zentral einen Einlass- bzw. Anschlussstutzen 6 auf, welcher mit einem zugehörigen Kanal, beispielsweise einem Saugkanal des Endoskops verbunden ist. Der Anschlussstutzen 6 steht mit dem Gehäuseinnenraum 8 des Ventilgehäuses 2 in Verbindung. Der Innenraum 8 weist eine zylindrische Form auf und ist zur zweiten Stirnseite 10 des Ventilgehäuses 2 hin geöffnet. Von der zweiten Stirnseite 10 her ist der Ventileinsatz 4 in das Ventilgehäuse 2 eingesetzt. In dem Innenraum 8 des Ventilgehäuses 2 ist im Bereich der der Stirnseite 10 abgewandten Seite am Umfang ein Absatz 12 ausgebildet, auf dem eine Dichtmembran 14 aufliegt. Der Ventileinsatz 4 weist ebenfalls eine zylindrische Außenkontur auf, welche dem Innendurchmesser des Ventiigehäuses 2 entspricht, so dass der Ventileinsatz 4 in das Ventilgehäuse 2 passend eingesetzt werden kann und in dem Ventilgehäuse 2 in radialer Richtung fixiert wird. Die dem Anschlussstutzen 6 zugewandte Seite des Ventileinsatzes 4 ist plan ausgebildet und liegt an der Dichtmembran 14 an. Der Dichteinsatz 4 weist im Bereich seines dem Anschlussstutzen 6 zugewandten Endes eine umlaufende Nut 16 auf, in die die Dichtmembran 14 mit ihrem umfänglichen Rand 18 eingreift. Die Dichtmembran 14 ist im Wesentlichen als plane, runde Scheibe mit einem Durchmesser entsprechend dem Innendurchmesser des Ventilgehäuses 2 ausgebildet. Am Umfang weist die Dichtmembran 14 einen vorstehenden Rand 18 auf, welcher mit einem radial nach innen gerichteten Vorsprung in die Umfangsnut 16 an dem Ventileinsatz 4 eingreift. Die Dichtmembran 14 kann daher auf dem Ventileinsatz 4 aufgeklipst bzw. aufgeschnappt werden, so dass sie von dem Ventileinsatz 4 gehalten wird und mit diesem eine Einheit bildet. Der Ventileinsatz 4 mit der Dichtmembran 14 ist so in das Ventilgehäuse 2 eingesetzt, dass die Dichtmembran 14 an ihrem Umfang vollständig an dem Absatz 12 in dem Ventilgehäuse 2 anliegt und somit den dem Anschlussstutzen 6 zugewandten Bereich des Innenraumes 8 des Ventilgehäuses 2 abdichtet.

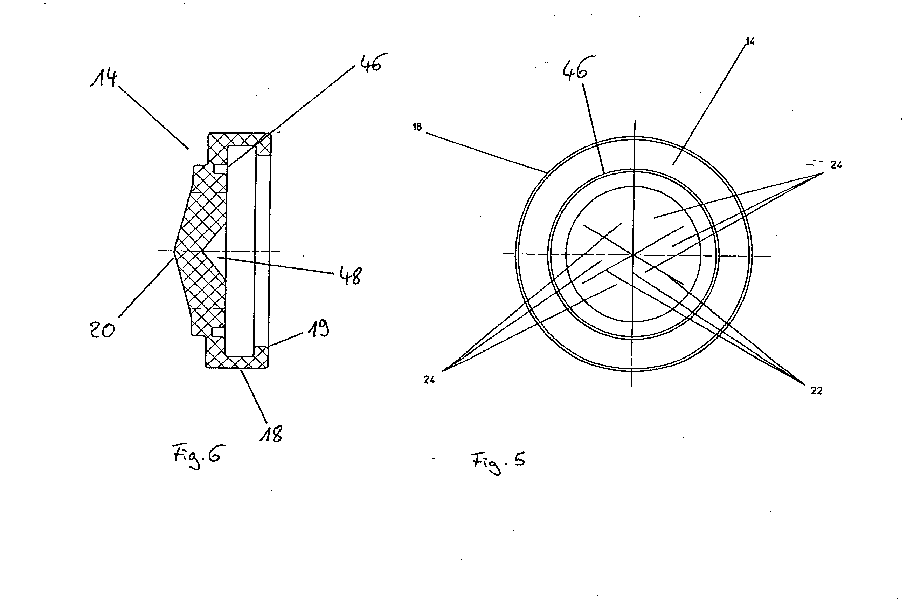

Auf ihrem dem Anschlussstutzen 6 des Ventilgehäuses 2 zugewandten Seite weist die Dichtmembran 14 eine kegelförmige Erhebung 20 auf, welche ihren höchsten Punkt bzw. ihre Spitze im Wesentlichen im Zentrum der Dichtmembran 14 hat. Wie in der Draufsicht der Dichtmembran 14 in Fig. 5 zu sehen ist, weist die Dichtmembran in ihrem Mittelbereich drei gekreuzte Schlitze 22 auf, welche ihren Kreuzungspunkt im Zentrum der Dichtmembran 14 haben. Die Schlitze 22 erstrecken sich durch die Dichtmembran 14 hindurch und verbinden die beiden Oberflächen der Dichtmembran 14 miteinander. Durch die gekreuzten Schlitze 22 werden vier dreieckige bzw. kreissegmentförmige Klappen 24 geschaffen, deren freie Enden bzw. Spitzen einander zugewandt sind. Diese Klappen 24 können jeweils um eine gedachte Achse, welche zwei benachbarte Endpunkte der Schlitze 22 miteinander verbindet, aufklappen, um eine Öffnung im Zentrum der Dichtmembran 14 freizugeben und das Ventil zu öffnen. In der gezeigten Ausführungsform sind drei gekreuzte Schlitze 22 vorgesehen, es können jedoch auch lediglich zwei oder mehr als drei gekreuzte Schlitze 22 vorgesehen werden, wobei sich die Zahl der Klappen 24 entsprechend ändert.

Der Ventileinsatz 4 ist im Wesentlichen rohrförmig ausgestaltet, wobei der Innenraum des Ventileinsatzes 4 einen kreisförmigen Querschnitt aufweist. Im Inneren des Ventileinsatzes 4 ist ein Ventilstößel 26 beweglich geführt. Der Ventilstößel 26 kann in Längsrichtung des Ventils, d. h. in Richtung der Längs- bzw. Mittelachse des Ventileinsatzes 2 in Richtung auf den Anschlussstutzen 6 hin verschoben werden. Der Ventilstößel 26 ist ebenfalls rohrförmig ausgebildet und weist an seinem dem Anschlussstutzen 6 zugewandten Ende eine Eintrittsöffnung 28 auf. Die diese Eintrittsöffnung 28 umgebende Kante des Ventilstößels 26 liegt an der Oberfläche der Dichtmembran 14 an. Ferner weist der Ventilstößel 26 radiale Öffnungen 30 auf, welche zum Innenraum des Ventileinsatzes 4 hin geöffnet sind. Der Innenraum des Ventileinsatzes 4 ist in seinem Mittelbereich erweitert, um einen Ringkanal zu bilden, welcher den Ventilstößel 26 umgibt. Dieser Ringkanal steht in Verbindung mit dem Auslassstutzen 32. Der Auslassstutzen 32 erstreckt sich radial zu dem Ventileinsatz 4 und dem Ventilgehäuse 2 in einer Richtung quer zu dem Einlassstutzen 6.

Das dem Anschlussstutzen 6 abgewandte Ende des Ventilstößels 26 ist als Druckkappe 34 ausgebildet. Die Druckkappe 34 erstreckt sich an dem dem Anschlussstutzen 6 abgewandten Ende über die gesamte Stirnseite des Ventileinsatzes 4, um eine große Druckfläche zur Betätigung des Ventils zu schaffen. Unterhalb der Druckkappe 34 ist ein Dichtring 36 angeordnet, welcher in geöffnetem Zustand des Ventils mit dem Ventileinsatz 4 zur Anlage kommt.

Der Ventileinsatz 4 ist von dem offenen Ende her in das napfförmige Ventilgehäuse 2 eingesetzt, wobei der Auslassstutzen 32 in eine Nut 38 in dem Ventilgehäuse 2 eingreift, um den Ventileinsatz in dem Ventilgehäuse 2 nach Art eines Bajonettverschlusses zu verriegeln und zu sichern. Der Auslassstutzen 32 ist vorzugsweise fest mit dem Ventileinsatz 4 verbunden oder einstückig mit diesem, beispielsweise aus Kunststoff, ausgebildet. Der Ventilstößel 26 wird durch umfängliche, sich radial erstreckende Vorsprünge 35 bzw. einen ringförmigen Vorsprung 35 an dem Ventilstößel 26 im Inneren des Ventileinsatzes 4 gehalten. Die Vorsprünge 35 sind auf den zwischen den radialen Öffnungen 30 verbleibenden Wandabschnitten bzw. Stegen angeordnet. Diese schmalen Stege zwischen den Öffnungen 30 sind elastisch nach innen auslenkbar, wobei die Vorsprünge radial nach innen bewegt werden. Dies ermöglicht, dass zum Entnehmen des Ventilstößels 26 die auf den Stegen angeordneten teilringförmigen Vorsprünge 35 durch die rampenförmigen Querschnittsverengungen 44 (siehe Fig. 2) zur Mittelachse hin ausgelenkt werden, sobald der Ventilstößel 26 aus dem Ventileinsatz 4 herausgezogen oder in diesen eingesetzt wird. Dabei ist die Federkraft der Stege, auf denen die Vorsprünge 35 angeordnet sind, zu überwinden. Vorzugsweise ist der Ventilstößel 26 ebenfalls aus Kunststoff ausgebildet und mit den Vorsprüngen 35 in den Ventileinsatz 4 eingerastet. Somit bilden Ventilstößel 26, Ventileinsatz 4 und die daran befestigte Dichtmembran 14 eine Baugruppe, welche leicht ausgetauscht und mit Hilfe des Auslassstutzens 32 in dem Ventilgehäuse 2 gehalten werden kann. Die Anzahl der für das Ventil erforderlichen Einzelteile wird dabei erheblich reduziert, so dass ein sehr kostengünstiges, leicht zu wartendes Ventil geschaffen wird, dessen Verschleißteile sehr einfach als zusammenhängende Baugruppe ersetzt werden können. Ebenfalls kann das Ventil zu Reinigungszwecken sehr leicht geöffnet oder zerlegt werden.

Ferner weist der Ventileinsatz 4 an seiner dem Anschlussstutzen 6 abgewandten Seite eine nach außen hin offene Umfangserweiterung auf, welche einen Ringraum 40 bildet, der den Ventilstößel 26 umgibt und mit den radialen Öffnungen 30 in dem Ventilstößel 26 in Verbindung steht. Auf diese Weise wird in geschlossenem Zustand des Ventils, welcher in Fig. 1 gezeigt ist, ein Strömungsweg von dem Auslassstutzen 32 durch die Öffnungen 30 und den Ringraum 40 zur Atmosphäre hin gebildet, um Luft, sogenannte "Falschluft", anzusaugen, während das Ventil bzw. die Dichtmembran 14 geschlossen ist. Dies ist zweckmäßig, wenn der Auslassstutzen 32 mit einer kontinuierlich wirkenden Unterdruckquelle verbunden ist.

Das Ventil weist in seinem geschlossenen Zustand eine gute Dichtwirkung auf, da die Dichtmembran 14 sowohl durch den im Ventileinsatz 4 erzeugten Unterdruck als auch die durch die elastische Ausgestaltung der Dichtmembran 14 hervorgerufene Rückstellkräfte in ihrer an der Stirnseite des Ventileinsatzes 4 anliegenden Lage gehalten wird. Dadurch werden die Schlitze 22 in der Dichtmembran 14 dicht geschlossen gehalten. Durch die kegelige Ausgestaltung der Dichtmembran 14 werden dabei große Anlageflächen bzw. Seitenflächen in den Schlitzen 22 geschaffen, die die Dichtwirkung und die Rückstellkraft der Dichtmembran 14 verbessern. Ein Umschlagen der beweglichen Klappen 24 der Dichtmembran 14 in das Innere des Ventileinsatzes 4 wird durch die Anlage der Dichtmembran 14 an der Umfangskante des Ventilstößels 26 und der Stirnseite des Ventileinsatzes 4 sowie durch die kegelige Ausgestaltung der Dichtmembran 14 selbst bei hohen Drücken sicher verhindert.

Fig. 2 zeigt den geöffneten Zustand des anhand von Fig. 1 beschriebenen Ventils. Zum Öffnen des Ventils wird die Druckkappe 34 mit dem Ventilstößel 26 weiter in den Ventileinsatz 4 hinein gedrückt in Richtung des Anschlussstutzens 6. Dabei kommt der Dichtring 36 unterhalb der Druckkappe 34 mit dem Ventileinsatz 4 zur Anlage und verschließt den Ringraum 40, so dass ein Ansaugen von Luft aus der Atmosphäre gestoppt wird.

An dem der Druckkappe 34 entgegengesetzten Ende drückt der Ventilstößel 26 gegen die beweglichen Klappen 24 der Dichtmembran 14, so dass diese in Richtung des Anschlussstutzens 6 hin aufklappen und eine Öffnung 42 in der Dichtmembran 14 freigeben. Zum leichteren Öffnen der Klappen 24 ist die die Öffnung 28 umgebende Umfangskante des Ventilstößels 26 an ihrer radial äußeren Seite angeschrägt bzw. angefast. Das Öffnen der Dichtmembran 14 gibt einen Strömungsweg durch die Öffnung 42, die Öffnung 28 sowie die Öffnungen 30 in dem Ventilstößel 26 zu dem Auslassstutzen 32 hin frei, so dass ein Fluid aus einem mit dem Anschlussstutzen 6 verbundenen Absaugkanal durch das Ventil hindurch abgesaugt werden kann.

Zum Schließen des Ventils wird die Druckkappe 34 losgelassen, wodurch sich der Ventilstößel 26 aufgrund der Rückstellkraft der elastisch ausgebildeten Dichtmembran 14 und des auf die Dichtmembran 14 wirkenden Unterdrucks in seine in Fig. 1 gezeigte Ausgangslage zurückbewegt. Dabei wird die Bewegung des Ventilstößels 36 durch die radialen Vorsprünge 35 begrenzt, welche an einer Querschnittsverengung 44 im Inneren des Ventileinsatzes 4 zur Anlage kommen.

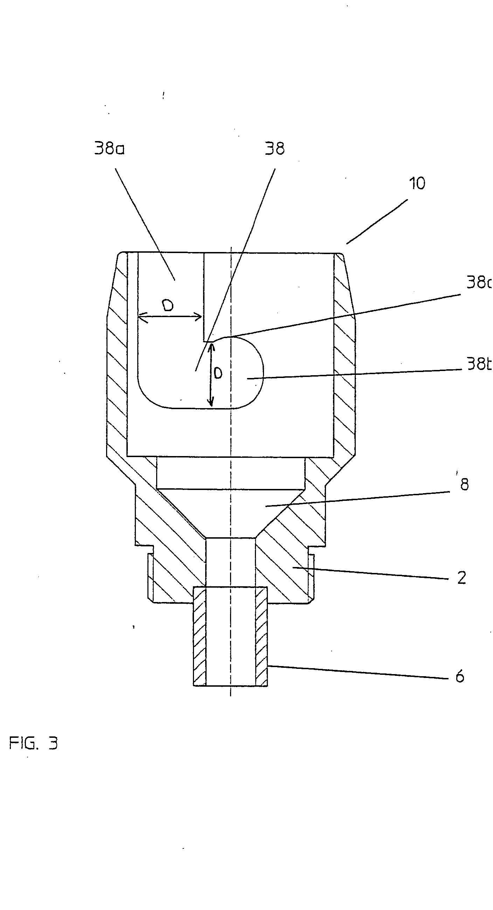

Fig. 3 zeigt eine Schnittansicht des Ventilgehäuses 2 gemäß Figuren 1 und 2. Am in Fig. 3 unteren Ende des Ventilgehäuses 2 ist der Anschlussstutzen 6 befestigt oder ausgebildet. Von dem in Fig. 3 oberen Ende her wird in das Ventilgehäuse 2 der in Fig. 3 nicht gezeigte Ventileinsatz 4 eingesetzt. In der Umfangswand des Ventilgehäuses 2 ist eine Nut 38 ausgebildet, welche zu der Stirnseite 10 hin geöffnet ist. Die Nut 38 weist zwei Schenkel 38a und 38b auf. Der Schenkel 38a erstreckt sich parallel zur Längsachse des Ventileinsatzes 2, d. h. senkrecht zu der Umfangskante an der Stirnseite 10 hin, und ist zur Stirnseite 10 hin geöffnet. Der Schenkel 38b erstreckt sich quer zu dem Schenkel 38a in Umfangsrichtung des Ventilgehäuses 2. Die Nut 38 mit den Schenkeln 38a und 38b weist eine Breite auf, welche dem Durchmesser D (siehe Fig. 2) des Auslassstutzens 32 entspricht. Dies erlaubt, dass der Auslassstutzen 32 beim Einsetzen des Ventileinsatzes 4 in das Ventilgehäuse 2 in der Nut 38 geführt wird. Beim Einsetzen des Ventileinsatzes 4 in das Ventilgehäuse 2 wird der Auslassstutzen 32 zunächst durch den Schenkel 38a der Nut 38 geführt, so dass der Ventileinsatz 4 nur in einer Richtung parallel zur Längsachse des Ventilgehäuses 2 in Richtung auf den Anschlussstutzen 6 bewegt werden kann. Wenn der Auslassstutzen 32 in den Bereich des quer verlaufenden Schenkels 38b der Nut 38 kommt, kann der Ventileinsatz 4 mit dem Auslassstutzen 32 in dem Ventilgehäuse 2 um die Längsachse des Ventilgehäuses 2 gedreht werden, wobei sich der Auslassstutzen 32 durch den Schenkel 38b der Nut 38 bewegt. Der Auslassstutzen 32 verschwenkt dabei in seiner Winkelposition bezüglich der Längsachse des Ventilgehäuses 2 und des Ventileinsatzes 4. Der quer verlaufende Schenkel 38b weist an seinem Ende eine Erweiterung 38c, insbesondere eine Ausbuchtung nach oben in Richtung parallel zu dem längs verlaufenden Schenkel 38a auf. Diese Ausbuchtung 38c dient zur Drehsicherung des Ventileinsatzes 4 mit dem Auslassstutzen 32. Beim Einsetzen des Ventileinsatzes 4 in das Ventilgehäuse 2 wird der Ventileinsatz 4 gegen die Dichtmembran 14 gedrückt, wobei diese elastisch gestaucht wird. Anschließend wird der Ventileinsatz 4 in dem Ventilgehäuse 2 verdreht, bis der Auslassstutzen 32 die Endposition des quer verlaufenden Nutschenkels 38b erreicht. In dieser Position wird der Druck auf den Ventileinsatz 4 gegen die Dichtmembran 14 gelöst, wodurch der Auslassstutzen 32 aufgrund der elastischen Rückstellkraft der Dichtmembran 14 in die Ausnehmung 38c am Ende der Nut 38b gedrückt wird. Durch den Eingriff des Auslassstutzens 32 in die Ausnehmung 38c ist der Ventileinsatz 4 gegen Verdrehen gesichert. Zum Lösen wird der Ventileinsatz 4 wieder gegen die Dichtmembran 14 gedrückt, so dass diese elastisch gestaucht wird, wobei der Auslassstutzen 32 von der Ausnehmung 38c außer Eingriff gebracht wird, so dass der Ventileinsatz wieder gedreht und dann aus dem Ventilgehäuse 2 entnommen werden kann.

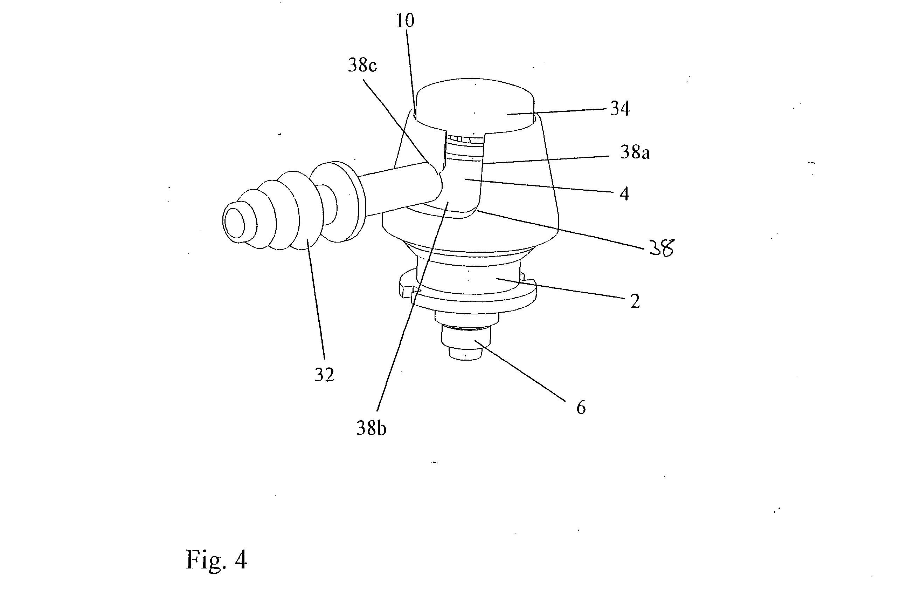

Fig. 4 veranschaulicht in einer perspektivischen Gesamtansicht des erfindungsgemäßen Ventils die zuvor beschriebene Sicherung des Ventileinsatzes 4 in dem Ventilgehäuse 2 nach Art eines Bajonettverschlusses. Wie anhand von Fig. 3 beschrieben, erstreckt sich der Schenkel 38a der Nut 38 parallel zur Längsachse des Ventils bzw. des Ventilgehäuses 2, während sich der zweite Schenkel 38b der Nut 38 in Umfangsrichtung durch die Wandung des Ventilgehäuses 2 erstreckt. Der Auslassstutzen 32 erstreckt sich durch die Nut 38 und sichert den Ventileinsatz 4 in axialer Richtung in dem Ventilgehäuse 2. Der Anschluss- bzw. Auslassstutzen 32 übernimmt somit eine Doppelfunktion, einerseits den Anschluss eines Absaugschlauches und andererseits die Sicherung des Ventileinsatzes 4 in dem Ventilgehäuse 2.

Fig. 6. zeigt eine Schnittansicht der in Fig. 5 gezeigten Dichtmembran 14. Die Dichtmembran 14 ist napfförmig ausgebildet, d. h. sie weist an ihrem äußeren Umfang einen senkrecht zur Fläche der Dichtmembran 14 vorstehenden Rand 18 auf. An der äußeren Kante weist der Rand 18 einen radial nach innen gerichteten Ringvorsprung 19 auf, welcher zur Befestigung der Dichtmembran 14 an dem Ventileinsatz 4 in die Umfangsnut 16 an dem Ventileinsatz 4 eingreift. Die einem Ventilstößel 26 abgewandten Seite, d. h. die dem Anschlussstutzen 6 zugewandte Seite der Dichtmembran 14 ist kegelförmig ausgebildet. Die an dieser Oberfläche der Dichtmembran 14 ausgebildete kegelförmige Verdickung 20 hat ihre Spitze bzw. ihren höchsten Punkt im Zentrum der Dichtmembran und im Wesentlichen im Schnittpunkt der Schlitze 22 (siehe Fig. 5). An der entgegengesetzten Oberfläche, d. h. der dem Ventilstößel 26 zugewandten Oberfläche, weist die in Fig. 6 gezeigte Dichtmembran 14 eine kegelförmige Ausnehmung 48 auf. Diese Ausgestaltung ist eine bevorzugte Ausführungsform, welche jedoch nicht zwingend ist; so weist die Dichtmembran 14 gemäß Figuren 1 und 2 keine solche Ausnehmung 48 auf. Diese Ausnehmung 48 bewirkt, dass im geöffneten Zustand der Klappen 24 (siehe Fig. 5) eine größere Durchtrittsöffnung geschaffen Wird, da weniger Material in den Strömungsweg hineinragt.

Ferner weist die Dichtmembran 14 gemäß Fig. 6 eine Ringnut 46 an der dem Ventileinsatz 4 zugewandten Oberfläche auf. Auch dies ist eine bevorzugte Ausgestaltung, welche bei der Dichtmembran gemäß Figuren 1 und 2 nicht vorgesehen ist. Die Ringnut 46 ist im Bereich des Umfangs an der dem Ventileinsatz 4 zugewandten Seite der Dichtmembran 14 vorgesehen, d. h. sie erstreckt sich in der Nähe des Randes 18 radial weiter innen liegend parallel zu diesem. Dabei verläuft die Ringnut 46, wie in Fig. 5 zu sehen ist, außerhalb der Schlitze 22, welche vollständig in dem von der Ringnut 46 umschlossenen Bereich angeordnet sind. Vorzugsweise sind dabei die Enden der Schlitze 22 radial nach innen von der Ringnut 46 beabstandet. Die Ringnut 46 dient dazu, die von den Schlitzen 22 definierten Klappen 24 von dem äußeren Randbereich der Dichtmembran 14 zu entkoppeln. Mit dem Randbereich wird die Dichtmembran 14 zwischen dem Ventileinsatz 4 und dem Ventilgehäuse 2 eingespannt bzw. eingeklemmt. Dabei werden Spannungen im Inneren der Dichtmembran 14 erzeugt. Die Ringnut 46 bewirkt nun, dass der zentrale von der Ringnut 46 umschlossene Bereich der Dichtmembran 14 von diesen Spannungen weitgehend freigehalten wird. Dadurch wird erreicht, dass die Klappen 24 beim Einklemmen der Dichtmembran 14 in dem Ventilgehäuse 2 nicht durch Spannungen beeinflusst werden. Somit kann sichergestellt werden, dass die Schlitze 22 und Klappen 24 ihre vordefinierte Form behalten und eine zuverlässige Dichtung und ein definiertes Öffnen der Dichtmembran 14 möglich ist.

### Bezugszeichenliste

- 2 -: Ventilgehäuse
- 4 -: Ventileinsatz
- 6 -: Anschlussstutzen
- 8 -: Inneraum
- 10 -: Stirnseite
- 12 -: Absatz
- 14 -: Dichtmembran
- 16 -: Nut
- 18 -: Rand
- 19 -: Ringvorsprung
- 20 -: kegelförmige Verdickung
- 22 -: Schlitze
- 24 -: bewegliche Klappen
- 26 -: Ventilstößel
- 28 -: Eintrittsöffnung
- 30 -: Öffnungen
- 32 -: Auslassstutzen
- 34 -: Druckkappe
- 35 -: Vorsprung
- 36 -: Dichtring
- 38 -: Nut
- 38a, 38b -: Nutschenkel
- 38c -: Aussparung
- 40 -: Ringraum
- 42 -: Öffnung
- 44 -: Querschnittsverengung
- 46 -: Ringnut
- 48 -: Ausnehmung

## Patentansprüche

1. Absaugventü für ein Endoskop, bei welchem ein Ventilgehäuse (2) mit einer Eintrittsöffnung (6) zum Verbinden mit einem Absaugkanal eines Endoskops vorgesehen ist, im Inneren des Ventilgehäuses (2) über der Eintrittsöffnung (6) eine geschlitzte Dichtmembran (14) angeordnet ist, die Dichtmembran (14) in dem Ventilgehäuse (2) durch einen Ventileinsatz (4) fixiert wird, in dem Ventileinsatz (4) ein quer zu der Dichtmembran (14) bewegbarer Ventilstößel (26) zum Öffnen der Dichtmembran (14) angeordnet ist, und der Ventileinsatz (4) kraft- und/oder formschlüssig in dem Ventilgehäuse (2) fixiert ist.

2. Absaugventil nach Anspruch 1, bei welchem der Ventileinsatz (4) einen Auslassstutzen (32) aufweist, welcher mit dem Ventilgehäuse (2) zur Fixierung des Ventileinsatzes (4) formschlüssig in Eingriff ist.

3. Absaugventil nach Anspruch 1 oder 2, bei welchem das Ventilgehäuse (2) einen zylindrischen Innenraum (8) aufweist, wobei die Eintrittsöffnung (6) an einer ersten Stirnseite des Ventilgehäuses (2) vorgesehen ist und die entgegengesetzte Stirnseite (10) des Ventilgehäuses (2) zur Aufnahme des Ventileinsatzes (4) offen ausgebildet ist, und der Ventileinsatz (4) eine zu dem Innenraum (8) korrespondierende, zylindrische Außenkontur aufweist.

4. Absaugventil nach Anspruch 3, bei welchem die Dichtmembran (14) eine dem Querschnitt des Innenraumes (8) entsprechende Außenkontur aufweist und sich quer zur Längsachse des Ventilgehäuses (2) erstreckt.

5. Absaugventil nach einem der vorangehenden Ansprüche, bei welchem die Dichtmembran (14) zumindest zwei sich kreuzende Schlitze (22) aufweist, welche sich in Dickenrichtung durch die Dichtmembran (14) hindurch erstrecken.

6. Absaugventil nach einem der vorangehenden Ansprüche, bei welchem die Dichtmembran (14) in ihrem Zentrum eine größere Dicke aufweist als in ihrem Umfangsbereich.

7. Absaugventil nach Anspruch 6, bei welcher zumindest der Mittelbereich der Dichtmembran (14) kegelförmig ausgebildet ist.

8. Absaugventil nach Anspruch 6 oder 7, bei welchem der Kreuzungspunkt der zumindest zwei Schlitze (22) in der Dichtmembran (14) in dem Bereich der größten Dicke der Dichtmembran (14) liegt.

9. Absaugventil nach einem der vorangehenden Ansprüche, bei welchem an der dem Ventilstößel (26) zugewandten Oberfläche der Dichtmembran (14) eine zentrische, vorzugsweise kegelförmige Ausnehmung (48) ausgebildet ist.

10. Absaugventil nach einem der vorangehenden Ansprüche, bei welchem die Dichtmembran (14) in ihrem Umfangsbereich zwischen einem Absatz (12) in dem Ventilgehäuse (2) und einer Stirnseite des Ventileinsatzes (4) eingeklemmt ist.

11. Absaugventil nach einem der vorangehenden Ansprüche, bei welchem an der dem Ventilstößel (26) zugewandten Oberfläche der Dichtmembran (14) eine Ringnut (46) ausgebildet ist, welche radial außerhalb der Schlitze (22) gelegen ist.

12. Absaugventil nach einem der Ansprüche 2 bis 11, bei welchem sich der Auslassstutzen (32) quer zur Längsachse des Ventilgehäuses (2) und der Eintrittsöffnung (6) erstreckt.

13. Absaugventil nach Anspruch 120, bei welchem in einer Wandung des Ventilgehäuses (2) eine l-förmige Nut (38) ausgebildet ist, deren erster Abschnitt (38a) sich im Wesentlichen parallel zur Längsachse des Ventilgehäuses (2) erstreckt und zum Rand (10) des Ventilgehäuses (2) hin geöffnet ist, und deren zweiter Abschnitt (38b) quer zu dem ersten Abschnitt (38a) im Wesentlichen in Umfangsrichtung des Ventilgehäuses (2) verläuft, wobei die Nut (38) eine Breite aufweist, welche der Querschnittsgröße des Auslassstutzens (32) entspricht.

14. Absaugventil nach einem der vorangehenden Ansprüche, bei welchem der Ventileinsatz (4) in Richtung der Längsachse des Ventilgehäuses (2) ein Durchgangsloch aufweist, in welchem der Ventilstößel (26) in Längsrichtung beweglich angeordnet ist, und eine Austrittsöffnung aufweist, welche sich in radialer Richtung zu dem Durchgangsloch hin öffnet, wobei der Ventilstößel (26) rohrförmig ausgebildet ist und an seiner der Dichtmembran (14) zugewandten Seite eine Eintrittsöffnung (28) und an seinem Umfang zumindest eine radiale Öffnung (30) aufweist, welche mit der Austrittsöffnung in Verbindung steht.

15. Absaugventil nach einem der vorangehenden Ansprüche, bei welchem die der Dichtmembran (14) abgewandte Stirnseite des Ventilstößels (26) als geschlossene Druckkappe (34) ausgebildet ist.

16. Absaugventil nach Anspruch 15, bei welchem zwischen der Druckkappe (34) und dem Ventileinsatz (4) ein Dichtelement (36) angeordnet ist, welches im geöffneten Zustand des Absaugventils das Durchgangsloch in dem Ventileinsatz (4) zu der Druckkappe (34) hin abdichtet.

17. Absaugventil nach einem der vorangehenden Ansprüche, bei welchem der Ventileinsatz (4) mit dem Ventilstößel (26) und der Dichtmembran (14) eine austauschbare Baugruppe bildet.

18. Absaugventil nach einem der vorangehenden Ansprüche, bei welchem die Bewegungsmöglichkeit des Ventilstößels (26) innerhalb des Ventileinsatzes (4) durch zumindest einen Anschlag (35) begrenzt ist.

19. Absaugventil nach Anspruch 18, bei welchem der zumindest eine Anschlag (35) auf einem an dem Ventilstößel (26) ausgebildeten elastischen Steg angeordnet ist.

20. Absaugventil nach einem der vorangehenden Ansprüche, bei welchem am Umfang des Ventileinsatzes (4) zumindest ein Vorsprung ausgebildet ist, welchen die Dichtmembran (14) umgreift.

21. Endoskop mit einem Absaugventil gemäß einem der vorangehenden Ansprüche, bei welchem das Ventilgehäuse (2) fest mit dem Endoskop verbunden ist.

22. Endoskop nach Anspruch 21, bei welchem das Ventilgehäuse (2) einstückig mit zumindest einem Bauteil des Endoskops ausgebildet ist.

23. Ventileinsatz (4) für ein Absaugventil, welcher ein in Längsrichtung verlaufendes Durchgangsloch, in dem ein in Längsrichtung beweglicher Ventilstößel (26) angeordnet ist, und einen sich radial erstreckenden Anschlussstutzen (32) aufweist.

24. Ventileinsatz nach Anspruch 23, bei welchem an einer Stirnseite des Ventileinsatzes (4) eine Dichtmembran (14) befestigt ist, welche durch Bewegung des Ventilstößels (26) geöffnet werden kann.

25. Ventileinsatz nach Anspruch 23 oder 24, bei welchem der Ventilstößel (26) rohrförmig ausgebildet ist und an einer Stirnseite eine Eintrittsöffnung (28) sowie an seinem Umfang zumindest eine radiale Austrittsöffnung (30) aufweist, welche mit dem Auslassstutzen (32) in Verbindung steht.

26. Ventileinsatz nach Anspruch 25, bei welchem die der Eintrittsöffnung (28) entgegengesetzte Stirnseite des Ventilstößels (26) als geschlossene Druckfläche (34) ausgebildet ist.
